# EUROPEAN PATENT SPECIFICATION

(11) **EP 2 690 097 B1**
(45) Date of publication and mention of the grant of the patent: **17.05.2017**
(21) Application number: 12763196.8
(22) Date of filing: 19.03.2012
(51) Int. Cl.: C07D 263/44

(54) **METHOD FOR CRYSTALLIZING GLUTAMIC ACID BENZYL ESTER N-CARBOXYLIC ANHYDRIDE**
VERFAHREN ZUR KRISTALLISATION VON GLUTAMINSÄURE-BENZYLESTER-N-CARBOXYL-ANHYDRID
PROCÉDÉ DE CRISTALLATION D'ANHYDRIDE N-CARBOXYLIQUE D'ESTER BENZYLIQUE D'ACIDE GLUTAMIQUE

(30) Priority: 25.03.2011 JP 2011068878
(43) Date of publication of application: 29.01.2014
(73) Proprietor: Nippon Soda Co., Ltd., Tokyo 100-8165 (JP)
(72) Inventor: SUMIYA, Hiroshi, Takaoka-shi Toyama 933-8507 (JP); IPPOSHI, Junji, Takaoka-shi Toyama 933-8507 (JP)
(74) Representative: Cabinet Plasseraud
(86) International application number: PCT/JP2012/057000
(87) International publication number: WO 2012/132991

(56) References cited:
- JP-A- 2005 154 768
- JP-A- 2008 518 032
- US-A1- 2002 082 431
- US-A1- 2002 183 551
- US-A1- 2006 106 229
- "Kapitel 2.2.42: Dichte von Feststoffen" In: "Europäisches Arzneibuch 4. Ausgabe Grundwerk 2002 Band 1", 1 January 2002 (2002-01-01), Deutscher Apotheker Verlag, Stuttgart, XP55111431, ISBN: 978-3-76-922947-9 pages 68-69, * page 69, Schütt- und Stampfdichte *
- E. R. BLOUT ET AL: "Polypeptides. III. The Synthesis of High Molecular Weight Poly-[gamma]-benzyl-L-glutamates 1", JOURNAL OF THE AMERICAN CHEMICAL SOCIETY, vol. 78, no. 5, 1 March 1956 (1956-03-01), pages 941-946, XP055129053, ISSN: 0002-7863, DOI: 10.1021/ja01586a020
- "Synthesis and Structural Study of the A-B-A Tri-Block Copolymer Consisting of Poly([gamma]-benzyl D,L-glutamate) or Poly([gamma]-methyl D,L-glutamate) as the A Component and Polybutadiene as the B Component", POLYMER JOURNAL, vol. 16, no. 10, 1 January 1984 (1984-01-01), pages 739-749, XP055129304,
- HYUNG W ET AL: "Drowning-out crystallization of l-proline: Effect of anti-solvent composition and processing parameters on crystal size and shape", POWDER TECHNOLOGY, ELSEVIER SEQUOIA, LAUSANNE, CH, vol. 186, no. 2, 11 August 2008 (2008-08-11), pages 137-144, XP022931880, ISSN: 0032-5910, DOI: 10.1016/J.POWTEC.2007.11.007 [retrieved on 2007-11-17]
- KITAMURA M ET AL: "Effect of Temperature on Antisolvent Crystallization and Transformation Behaviors of Thiazole-Derivative Polymorphs", CRYSTAL GROWTH & DESIGN, ACS, WASHINGTON, DC, US, vol. 6, no. 5, 1 January 2006 (2006-01-01) , pages 1214-1218, XP003020202, ISSN: 1528-7483, DOI: 10.1021/CG050635F

## Description

### TECHNICAL FIELD

The present invention relates to a method for crystallizing a glutamic acid benzyl ester N-carboxylic anhydride which is a useful compound, and crystal polymorphs that can be obtained by the method.

Priority is claimed on Japanese Patent Application No. 2011-068878, filed March 25, 2011.

### BACKGROUND ART

An N-carboxylic anhydride obtained from an α-amino acid is an extremely useful compound due to the activity of the acid group thereof. Several production methods of N-carboxylic anhydride are known, and purification methods performed by recrystallization of the compound are also known.

For example, there is a disclosure regarding a method of dissolving a coarse carboxylic anhydride, which is obtained by removing benzyl alcohol from glutamic acid benzyl ester N-benzyloxycarbonyl as a precursor by using phosphorus pentachloride and performing cyclization condensation, in ethyl acetate and adding carbon tetrachloride thereto to cause recrystallization (refer to NPL 1). Regarding the recrystallization method, the disclosure merely describes the solvent used and does not specifically describe the crystallization temperature. Moreover, a bulk density of the crystals obtained in this manner is 0.23 g/cm³, which is an extremely low value.

In addition, there is a disclosure regarding a recrystallization method that repeats 6 times an operation in which a coarse glutamic acid benzyl ester N-carboxylic anhydride is obtained by cyclizing γ-benzyl-L-glutamate by using triphosgene, the coarse crystals are dissolved in ethyl acetate, and hexane is added thereto to cause crystallization (refer to PTL 1). Regarding the recrystallization method, the disclosure merely describes the solvent used and does not specifically describe the crystallization temperature. Moreover, a bulk density of the crystals obtained in this manner is 0.38 g/cm³, which is an extremely low value.

Further, there is a disclosure regarding a process in which γ-benzyl-L-glutamate is suspended in ethyl acetate, phosgene gas is blown into the suspension under cooling, the mixture is heated at 60°C and then reacted for 3 hours under reduced pressure so as to be distilled under reduced pressure, and heptane that is in almost the same amount as that of the mixture is added thereto under a heating condition, the mixture is cooled to 0°C, and the precipitated crystals are filtered to obtain a target carboxylic anhydride (refer to PTL 2). It is considered that in a general recrystallization operation, crystals are precipitated not in a state where heptane as an anti-solvent is added under a heating condition, but in a state of performing cooling at 0°C. Moreover, PTL 2 does not describe what type of crystal polymorph the obtained crystals have or the degree of the bulk density.

There is another disclosure regarding a process in which γ-benzyl-L-glutamate is reacted with phosgene in tetrahydrofuran (refer to PTL 3, example 2). The tetrahydrofuran and excess phosgene were removed by vacuum stripping whereupon the concentrated residue crystallized. The stripped residue was dissolved in 2.8 liters anhydrous ethyl acetate and the cloudy mixture vacuum filtered to remove insoluble solids. Anhydrous hexane (5.5 liters) were added gradually with stirring to the filtrate to crystallize the product. The product slurry was stirred for 30 minutes and refrigerated at 5°C overnight and the product was isolated by filtration.

### Citation List

### Patent Literature

[PTL 1] Japanese Unexamined Patent Application, First Publication No. 2005-154768
[PTL 2] Japanese Unexamined Patent Application, First Publication No. 2002-371070
[PTL 3] US Patent Application No. 2006/0106229

### Non-Patent Literature

[NPL 1] J. Chem. Soc., 1950, 3239

### DISCLOSURE OF INVENTION

### Problems to be Solved by the Invention

Conventionally, regarding a glutamic acid benzyl ester N-carboxylic anhydride, crystals having a high bulk density are not known. Moreover, there is a demand for a crystal polymorph of glutamic acid benzyl ester N-carboxylic anhydride that is excellent in storage stability.

An object of the present invention is to provide a glutamic acid benzyl ester N-carboxylic anhydride that has a high bulk density and is excellent in storage stability. Means for Solving the Problems

The present inventors conducted thorough research to solve the above problems. As a result, they found that the crystals, which are precipitated at a temperature of equal to or higher than a certain degree by means of adding an anti-solvent in an amount of equal to or larger than a certain degree to a good solvent in an amount of equal to or larger than a certain degree, become crystals having a high bulk density, thereby completing the present invention.

That is, the present disclosure relates to a glutamic acid benzyl ester N-carboxylic anhydride which includes crystal polymorphs (crystals A) having peaks at 6.5°, 13.0°, and 19.5° at an angle of diffraction (2 θ°) in a powder X-ray diffraction diagram using CuKα rays as an X-ray source and has a bulk density of 0.45 g/cm³ or higher.

It is preferable that the glutamic acid benzyl ester N-carboxylic anhydride include the crystals A, in which the crystals A differ from each other in terms of preferred orientation and have peaks at 15.0°, 17.3°, 18.9°, 19.9°, 21.2°, 23.2°, 23.9°, 25.0°, and 27.7° at an angle of diffraction (2 θ°) in a powder X-ray diffraction diagram using CuKα rays as an X-ray source.

The present invention relates to a method for crystallizing a glutamic acid benzyl ester N-carboxylic anhydride, including dissolving glutamic acid benzyl ester N-carboxylic anhydride in a solvent which has been heated at a temperature equal to or higher than 40°C and lower than a boiling point thereof and is in an amount of 0.5 L or more per mol of the glutamic acid benzyl ester N-carboxylic anhydride, adding an anti-solvent which is in an amount of 1.4 L or more per mol of the glutamic acid benzyl ester N-carboxylic anhydride at a temperature equal to or higher than 40°C and lower than a boiling point thereof, precipitating crystals at a temperature equal to or higher than 40°C and lower than a boiling point thereof, and cooling the crystals.

The solvent heated at a temperature equal to or higher than 40°C and lower than a boiling point thereof is ethyl acetate, the anti-solvent is heptane, hexane, pentane, or petroleum ether. In addition, in the above crystallization method of a glutamic acid benzyl ester N-carboxylic anhydride, a bulk density of the obtained crystals is preferably 0.45 g/cm³ or higher.

### Effects of the Invention

If the crystallization method of the present invention is used, crystal polymorphs excellent in storage stability can be obtained.

### BRIEF DESCRIPTION OF THE DRAWINGS

FIG. 1 is a result obtained by powder X-ray crystal analysis performed on a glutamic acid benzyl ester N-carboxylic anhydride obtained in Example 1.
FIG. 2 is a result obtained by powder X-ray crystal analysis performed on a glutamic acid benzyl ester N-carboxylic anhydride obtained in Example 2.
FIG. 3 is a result obtained by powder X-ray crystal analysis performed on a glutamic acid benzyl ester N-carboxylic anhydride obtained in Example 3.
FIG. 4 is a result obtained by powder X-ray crystal analysis performed on a glutamic acid benzyl ester N-carboxylic anhydride obtained in Comparative example 1.
FIG. 5 is a result obtained by powder X-ray crystal analysis performed on a glutamic acid benzyl ester N-carboxylic anhydride obtained in Comparative example 2.

### BEST MODE FOR CARRYING OUT THE INVENTION

In the crystallization method of the present invention, a glutamic acid benzyl ester N-carboxylic anhydride is dissolved in a solvent which has been heated at a temperature equal to or higher than 40°C and lower than a boiling point thereof and is in an amount of 0.5 L or more per mol of the glutamic acid benzyl ester N-carboxylic anhydride, an anti-solvent which is in an amount of 1.4 L or more of the anti-solvent per mol of the glutamic acid benzyl ester N-carboxylic anhydride is added thereto at the same temperature so as to precipitate crystals at the same temperature, and the crystals are cooled. The temperature for cooling after heating is not particularly limited, but is preferably 0°C to room temperature and more preferably in a range of from 3°C to 10°C.

The solvent used dissolves a glutamic acid benzyl ester N-carboxylic anhydride. The solvent is a polar solvent selected from ethyl acetate. Compounds having an active hydrogen atom, such as alcohols, are not preferable since these react with a substrate.

The anti-solvent used is not particularly limited as long as the solubility of a glutamic acid benzyl ester N-carboxylic anhydride is low. The anti-solvent is an aliphatic hydrocarbon selected from heptane, hexane, pentane, or petroleum ether. Two or more kinds of these may be used by being mixed with each other.

For dissolving a glutamic acid benzyl ester N-carboxylic anhydride in a solvent at a temperature equal to or higher than 40°C and lower than a boiling point thereof, the glutamic acid benzyl ester N-carboxylic anhydride may be heated after being added to the heated solvent, or the glutamic acid benzyl ester N-carboxylic anhydride may be added to the solvent having not yet been heated. After the glutamic acid benzyl ester N-carboxylic anhydride dissolves completely, an anti-solvent is added thereto at a temperature kept to be equal to or higher than 40°C and lower than a boiling point thereof, and crystals are precipitated at a temperature equal to or higher than 40°C and lower than a boiling point thereof. The amount of the solvent for dissolving the glutamic acid benzyl ester N-carboxylic anhydride is 0.5 L or more based on 1 mol of the glutamic acid benzyl ester N-carboxylic anhydride. The amount of the anti-solvent used is sufficient for precipitating crystals. The amount of anti-solvent is 1.4 L or more based on 1 mol of the glutamic acid benzyl ester N-carboxylic anhydride.

The method of adding an anti-solvent is not particularly limited as long as the temperature of the solution can be maintained within the above range. Specifically, the anti-solvent may be added dropwise little by little, or a method of adding it by a certain amount may be used.

Moreover, as another crystallization method not according to the invention, if a method of dissolving glutamic acid benzyl ester N-carboxylic anhydride under heating by using chloroform as a solvent and cooling the resultant to cause crystallization is used, a target crystal polymorph having a high bulk density can be obtained.

The amount of chloroform used is not particularly limited as long as the amount is within a range in which general recrystallization occurs, and the amount is preferably within a range of from 1.5 L to 3.0 L and more preferably within a range of from 1.8 L to 2.2 L, based on 1 mol of the glutamic acid benzyl ester N-carboxylic anhydride.

If the crystallization method of the present invention is used, it is possible to obtain crystal polymorphs (crystal A) having peaks at 6.5°, 13.0°, and 19.5° at an angle of diffraction (2 θ°) in a powder X-ray diffraction diagram and having a bulk density of 0.45 g/cm³ or higher. In addition, the crystals A may include crystals A which differ from each other in terms of preferred orientation and have peaks at 15.0°, 17.3°, 18.9°, 19.9°, 21.2°, 23.2°, 23.9°, 25.0°, and 27.7° at an angle of diffraction (2 θ°) in a powder X-ray diffraction diagram.

### Examples

Hereinafter, the present invention will be described in more detail based on examples, but the present invention is not limited to the examples.

### Example 1

237.0 g (1.0 mol) of L-glutamic acid benzyl ester N-carboxylic anhydride was dissolved in 1,315 ml (1.3 L/mol) of ethyl acetate, and the internal pressure of the reaction system was reduced to be slightly lower than atmospheric pressure. In this state, 137.0 g (1.4 equivalents) of phosgene gas was blown into the reaction system while the temperature thereof was being raised to 60°C from room temperature over 1 hour, and 518.0 g (5.2 equivalents) of phosgene gas was further blown into the reaction system for 3 hours and 45 minutes at 60°C to cause a reaction. After the reaction ended, nitrogen was injected into the system to remove phosgene, and then for adjusting concentration, ethyl acetate and phosgene were distilled away under reduced pressure until the concentration thereof became 0.5 L/mol, followed by heating at 60°C. Thereafter, while the temperature thereof was being maintained at 60°C, 1,340 ml (1.4 L/mol) of heptane was added dropwise thereto over 1 hour and 20 minutes to precipitate crystals. The obtained crystals were cooled at 5°C or a lower temperature and then filtered, followed by drying under reduced pressure, thereby obtaining 233.99 g (yield of 89%) of a target L-glutamic acid benzyl ester N-carboxylic anhydride.

26.5 g (0.1 mol) of the obtained L-glutamic acid benzyl ester N-carboxylic anhydride was suspended in 50 ml (0.5 L/mol) of ethyl acetate and dissolved by increasing the temperature to 60°C. Subsequently, while the same temperature was being maintained, 140 ml (1.4 L/mol) of heptane was added thereto to precipitate crystals. The obtained crystals were cooled at 5°C or a lower temperature and then filtered, followed by drying under reduced pressure, thereby obtaining 24.9 g (yield of 94%) of a target L-glutamic acid benzyl ester N-carboxylic anhydride.

The obtained crystals were tap-filled in a cylindrical container having an inner diameter of 1.45 cm. At this time, a bulk density of the crystals was 0.50 g/cm³.

### <Method of powder X-ray crystal analysis>

The crystals were filled in a sample filling portion of a glass test plate and measured with a powder X-ray diffractometer (manufactured by Spectris Co., Ltd.: X'PertPro) (X-ray source: CuKα, output: 1.8 kW (45 kV-40 mA), measurement range: 2 θ = 4° to 60°).

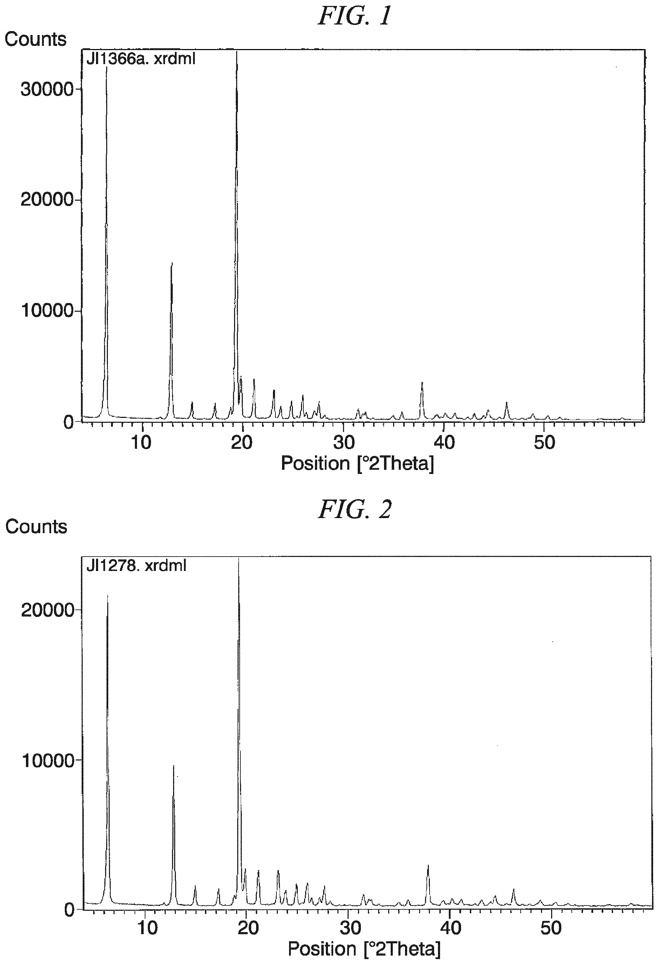

From FIG. 1, it was understood that the obtained crystals are crystal polymorphs of a glutamic acid benzyl ester N-carboxylic anhydride that include crystal polymorphs (crystals A) having peaks at 6.5°, 13.0°, and 19.5°at an angle of diffraction (2 θ°) and crystals A which differ from each other in terms of preferred orientation and have peaks at 15.0°, 17.3°, 18.9°, 19.9°, 21.2°, 23.2°, 23.9°, 25.0°, and 27.7°.

### Example 2

10.0 g (0.04 mol) of the L-glutamic acid benzyl ester N-carboxylic anhydride obtained by the same crystallization method as in Example 1 was suspended in 38 ml (1.0 L/mol) of ethyl acetate and dissolved by increasing the temperature thereof to 40°C. Thereafter, while the same temperature was being maintained, 106 ml (2.8 L/mol) of heptane was added thereto to precipitate crystals. The obtained crystals were cooled to room temperature and then filtered, followed by drying under reduced pressure, thereby obtaining 9.3 g (yield of 93%) of a target L-glutamic acid benzyl ester N-carboxylic anhydride.

FIG. 2 shows the result of powder X-ray crystal analysis that was performed on the obtained crystals under the same conditions as in Example 1. In addition, a bulk density of the crystals was 0.47 g/cm³.

### Example 3(not according to the invention)

5.0 g (0.02 mol) of the L-glutamic acid benzyl ester N-carboxylic anhydride obtained by the same crystallization method as in Example 1 was suspended in 38 ml (2.0 L/mol) of chloroform and dissolved by increasing the temperature thereof to 60°C, and then the solution was cooled to precipitate crystals. The obtained crystals were cooled to 5°C or a lower temperature and then filtered, followed by drying under reduced pressure, thereby obtaining 3.3 g (yield of 65%) of a target L-glutamic acid benzyl ester N-carboxylic anhydride.

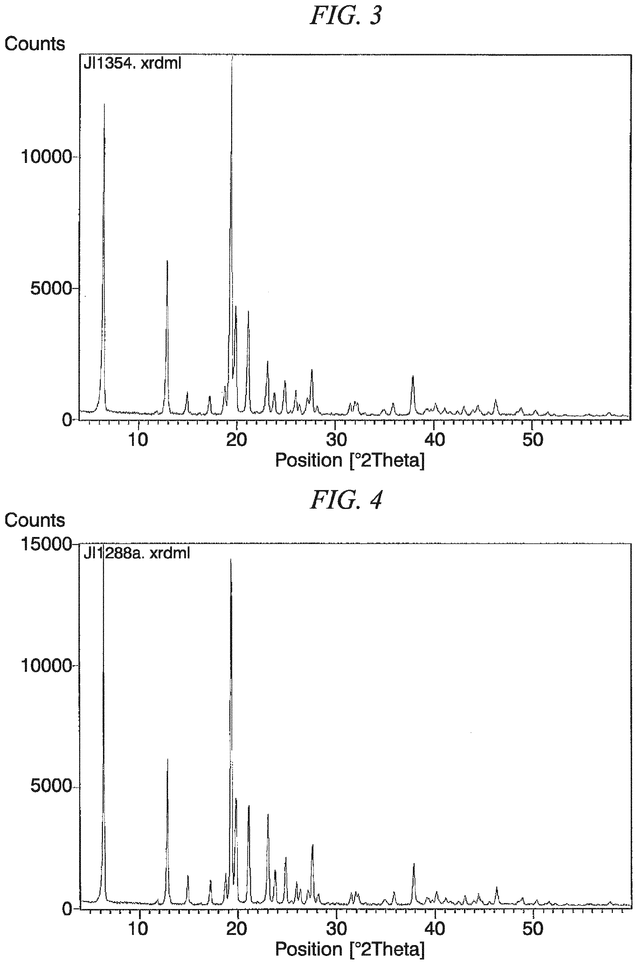

FIG. 3 shows the result of powder X-ray crystal analysis that was performed on the obtained crystals under the same conditions as in Example 1. In addition, a bulk density of the crystals was 0.50 g/cm³.

### Comparative example 1

10.0 g (0.04 mol) of the L-glutamic acid benzyl ester N-carboxylic anhydride obtained by the same crystallization method as in Example 1 was suspended in 27 ml (0.7 L/mol) of ethyl acetate and dissolved by increasing the temperature thereof to 70°C. Thereafter, while the same temperature was being maintained, 53 ml (1.4 L/mol) of heptane was added thereto, and the solution was cooled to precipitate crystals. The obtained crystals were cooled to 5°C or a lower temperature and then filtered, followed by drying under reduced pressure, thereby obtaining 9.4 g (yield of 94%) of a target L-glutamic acid benzyl ester N-carboxylic anhydride.

FIG. 4 shows the result of powder X-ray crystal analysis that was performed on the obtained crystals under the same conditions as in Example 1. In addition, a bulk density of the crystals was 0.43 g/cm³.

### Comparative example 2

14.0 g (0.05 mol) of the L-glutamic acid benzyl ester N-carboxylic anhydride obtained by the same crystallization method as in Example 1 was suspended in 389 ml (7.4 L/mol) of methyl-tert-butyl ether and dissolved by increasing the temperature thereof to 55°C, and then the solution was cooled to precipitate crystals. The obtained crystals were cooled to 5°C or a lower temperature and then filtered, followed by drying under reduced pressure, thereby obtaining 9.4 g (yield of 67%) of a target L-glutamic acid benzyl ester N-carboxylic anhydride.

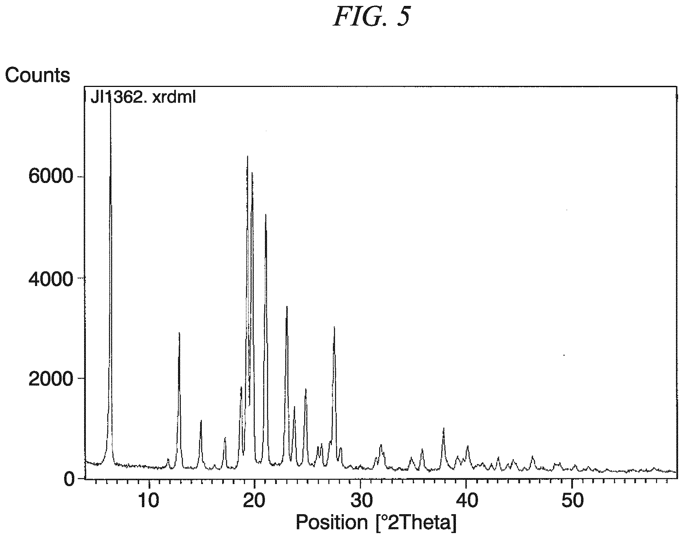

FIG. 5 shows the result of powder X-ray crystal analysis that was performed on the obtained crystals under the same conditions as in Example 1. In addition, a bulk density of the crystals was 0.29 g/cm³.

### [Test for storage stability]

About 0.4 g of the crystals obtained in Examples 1 and 2 and Comparative examples 1 and 2 was put into a cylindrical container and dried under reduced pressure in a desiccator for 6 hours. Subsequently, the pressure thereof was returned to normal pressure by using air with a temperature of 23°C and a humidity of 41%, and the desiccator in an air atmosphere was placed in a constant-temperature bath at 20°C to compare the storage stability of the crystals. Table 1 shows the residual rate of the L-glutamic acid benzyl ester N-carboxylic anhydride after 7 days.

[Table 1]

**Table 1 Test results**

| | Bulk density (g/cm³) | Residual rate (%) |
|---|---|---|
| Example 1 | 0.50 | 98, 99^{*1} |
| Example 2 | 0.47 | 97^{*1} |
| Comparative example 1 | 0.43 | 88 |
| Comparative example 2 | 0.29 | 54 |

| | | |
|---|---|---|
| *1 Indicates the result obtained when the atmosphere was substituted with air with a temperature of 23°C and a humidity of 31%. | | |

From the above result, it was understood that storage stability is excellent in crystal polymorphs having a bulk density of 0.45 g/cm³ or higher.

### INDUSTRIAL APPLICABILITY

If the crystallization method of the present invention is used, crystal polymorphs excellent in storage stability can be obtained. Accordingly, the present application is extremely useful in the industrial field.

## Claims

1. A method for crystallizing a glutamic acid benzyl ester N-carboxylic anhydride, comprising:
dissolving glutamic acid benzyl ester N-carboxylic anhydride in a solvent which has been heated at a temperature equal to or higher than 40°C and lower than a boiling point thereof and is in an amount of 0.5 L or more per mol of the glutamic acid benzyl ester N-carboxylic anhydride;
adding an anti-solvent which is in an amount of 1.4 L or more per mol of the glutamic acid benzyl ester N-carboxylic anhydride at a temperature equal to or higher than 40°C and lower than a boiling point thereof;
precipitating crystals at a temperature equal to or higher than 40°C and lower than a boiling point thereof; and
cooling the crystals,
wherein the solvent that has been heated to a temperature equal to or higher than 40°C and lower than a boiling point thereof is ethyl acetate, and
wherein the anti-solvent is heptane, hexane, pentane, or petroleum ether.

## Patentansprüche

1. Verfahren zur Kristallisation eines Glutaminsäure-Benzylester-N-Carboxyl-Anhydrids, welches umfasst:
Lösen eines Glutaminsäure-Benzylester-N-Carboxyl-Anhydrids in einem Lösungsmittel, welches auf eine Temperatur von 40° C oder höher und niedriger als dessen Siedepunkt erwärmt worden ist und in einer Menge von 0,5 1 oder mehr pro Mol Glutaminsäure-Benzylester-N-Carboxyl-Anhydrid vorliegt;
Hinzufügen eines Antisolvents, welches in einer Menge von 1,41 oder mehr pro Mol Glutaminsäure-Benzylester-N-Carboxyl-Anhydrid bei einer Temperatur von 40°C oder höher und niedriger als dessen Siedepunkt vorliegt;
Ausfällen von Kristallen bei einer Temperatur von 40°C oder höher und niedriger als deren Siedepunkt; und
Abkühlen der Kristalle,
wobei das Lösungsmittel, welches auf eine Temperatur von 40°C oder höher und
niedriger als dessen Siedepunkt erwärmt worden ist, Ethylacetat ist, und
wobei das Antisolvent Heptan, Hexan, Pentan oder Petrolether ist.

## Revendications

1. Procédé de cristallisation d'un anhydride N-carboxylique d'ester benzylique d'acide glutamique, comprenant :
la dissolution d'anhydride N-carboxylique d'ester benzylique d'acide glutamique dans un solvant qui a été chauffé à une température supérieure ou égale à 40 °C et
inférieure à un point d'ébullition de celui-ci et est en une quantité de 0,5 L ou plus par mole de l'anhydride N-carboxylique d'ester benzylique d'acide glutamique ;
l'ajout d'un anti-solvant qui est en une quantité de 1,4 L ou plus par mole de l'anhydride N-carboxylique d'ester benzylique d'acide glutamique à une température supérieure ou égale à 40 °C et inférieure au point d'ébullition de celui-ci ;
la précipitation de cristaux à une température supérieure ou égale à 40 °C et
inférieure à un point d'ébullition de celui-ci ; et
le refroidissement des cristaux,
dans lequel le solvant qui a été chauffé à une température supérieure ou égale à 40 °C et inférieure à un point d'ébullition de celui-ci est l'acétate d'éthyle, et
dans lequel l'anti-solvant est l'heptane, l'hexane, le pentane ou un éther de pétrole.
